# EUROPEAN PATENT SPECIFICATION

(11) **EP 1 341 891 B1**
(45) Date of publication and mention of the grant of the patent: **02.03.2005**
(21) Application number: 01991802.8
(22) Date of filing: 03.12.2001
(51) Int. Cl.: C11D 3/37

(54) **TEXTILE CARE COMPOSITION**
TEXTILPFLEGEMITTEL
COMPOSITION D'ENTRETIEN DES TEXTILES

(30) Priority: 11.12.2000 GB 0030177
(43) Date of publication of application: 10.09.2003
(73) Proprietor: UNILEVER N.V., 3013 AL Rotterdam (NL); UNILEVER PLC, London EC4P 4BQ (GB)
(72) Inventor: OAKES, John, Unilever Research Port Sunlight, Wirral, Merseyside CH63 3JW (GB); SUGDON, Matthew, Basingstoke, Hampshire RG24 7BQ (GB)
(74) Representative: Elliott, Peter William
(86) International application number: PCT/EP2001/014379
(87) International publication number: WO 2002/048304

(56) References cited:
- WO-A-00/40210
- WO-A-01/59053
- US-A- 3 694 258
- US-A- 4 311 760
- US-A- 4 895 917

## Description

### Technical Field

This invention relates to textile care compositions and, in particular, to the use of the textile care compositions in a domestic laundering process to reduce fibre damage and/or improve textile dimensional stability.

### Background and Prior Art

The laundry process generally has several benefits for textiles, the most common being to remove dirt and stains from the textile during the wash cycle and to soften the textile during the rinse cycle. However, there are numerous disadvantages associated with repeated use of conventional laundry treatment compositions and/or the actual laundry process; one of these being a fairly harsh mechanical and/or chemical treatment of textiles in the laundry process.

Textiles can be damaged in several ways as a result of repeated laundering and/or wear. Textile pilling and loss of textile surface appearance e.g. fuzzing, shrinkage (or expansion), loss of colour from the textile or running of colour on the textile (usually termed 'dye transfer') are some of the common problems associated with repeated laundering. Loss of colour is generally believed to be due to one or more of three causes: dye loss, loss of dye fixative or bleaching of coloured components. These problems will occur from repeated hand washing as well as the more vigorous machine washing process.

The present invention is directed towards alleviating one or more of the problems referred to hereinabove.

A wide range of substantive polymeric materials have been proposed for use in laundry detergent compositions. For example, polyamide-polyamine textile treatment agents are described in WO 98/29530. The compositions are claimed to impart improved overall appearance to textiles laundered using the detergent compositions, in terms of surface appearance properties such as pill/fuzz reduction and anti-fading. Laundry compositions containing polyamide-polyamine treatment agents of similar types are taught in WO 97/42287. WO 96/15309 and WO 96/15310 describe anti-wrinkle compositions which contain a silicone and a film-forming polymer. An industrial process for treating fibres is disclosed in US 3949014, which describes the use of a polyamine-epichlorohydrin resin in a binder, together with an amphoteric high molecular weight compound having at least 2 cationic groups and at least 2 anionic groups per molecule. Methods for treating wool with compositions containing an amino functional polymer and a silicone polymer so as to impart shrink resistance are also known. US 4371517 discloses compositions for treating fibrous materials which contain cationic and anionic polymer useful in a non-domestic treatment, whereby the compositions increase the rigidity of cotton textile materials. Co-emulsifiers, for use in textile softener and other compositions, which contain cationic quaternary amine polymers, are taught in DD 221922.

From the above it can be seen that the mechanism of bonding of the beneficial polymer to the textile has largely been through an ionic interaction. Typically, the nitrogen containing structures of the polymer acquire a charge which results in binding to the textile. Other polymers make use of sulphate or thiosulphate groups to ensure ionic binding to a textile.

Cotton and wool differ markedly in their chemical properties. Cotton is a cellulosic material and is almost exclusively made of carbon, oxygen and hydrogen arranged in the familiar cellulose structure. In contrast, wool is a keratinaceous material and consequently contains a significant proportion of nitrogen and sulphur.

The presence of sulphur in wool has been exploited to covalently bind polymeric materials to its surface. US 3694258 discloses how thiourea can be reacted with epihalohydrins to convert the halo groups into the corresponding isothiouronium salts of which a range is disclosed. These salts can be used to treat wool, or blends of wool with other fibres. It is believed that these materials function by loss of the relatively labile urea group exposing a reactive sulphhydryl group which subsequently cross-links with the sulphur-containing groups present in the wool (typically as -SH). This covalently binds the polymer to the wool imparting a shrink resistance. US 3594355 teaches a similar process whereas US A-5254335 discloses an analogous process being used to improve the condition of hair (also a keratinaceous material). Further examples of the uses of isothiouronium salts in hair treatment compositions are found in US 5160733.

US 4895917 (Gruning) discloses organopolysiloxanes with Bunte salt groups and their use for the finishing of fabrics. Bunte salts have a terminal -S₂O₃ ⁻Na⁺ group. Under aqueous **acid** conditions this group is hydrolysed to the corresponding thiol. Under the **alkaline** conditions found in domestic washing machines, Bunte salts form the corresponding disulphides rather than thiols.

### Brief Description of the Invention

The present invention is based on the surprising finding that certain types of thiol-containing polymer can alleviate fibre damage in and/or the dimensional instability (e.g. shrinkage) of textiles which comprise cellulosic fibres such as cotton, for example. The term "dimensional stability", and related terms, used herein covers not only shrinkage of textiles but also shape retention, bagginess reduction and additionally, although less preferred, crease/wrinkle resistance in textiles. The term 'fibre damage' is intended to embrace colour loss.

According to the present invention, there is provided a process for the treatment of non-keratinaceous textiles which comprises the step of treating the textiles with a composition comprising:
a) a polymer comprising at least one protected thiol group, wherein the protecting group is labile under domestic washing conditions of pH from 8 to 11 and a temperature of from 10 to 80° Celsius, and
b) a textile compatible carrier,
   whereby under said domestic washing conditions, the polymer forms reactive thiol groups which cause covalent cross-linking of the polymer.

Without being limited by any theory of operation, it is believed that the upon loss of the protecting group the thiol groups cross-link without reaction with the textile. It is believed that this forms a structure which restricts relative movement of the textile fibres and consequently reduces damage to the textile fibres during the laundering process. It is believed that this reduces pilling and provides shrink resistance.

It is further believed that the structure prevents apparent colour loss by retarding damage to the fibres leading to a rough fibre surface which would give the appearance of colour loss due to a modification of the manner in which light is scattered from the fibre surface.

The textiles which may be treated in the present invention preferably comprise cellulosic fibres, preferably from 1% to 100% cellulosic fibres (more preferably 5% to 100% cellulosic fibres, most preferably 40% to 100%). When the textile contains less than 100% cellulosic fibres, the balance comprises other fibres or blends of fibres suitable for use in garments such as polyester, for example. Preferably, the cellulosic fibres are of cotton or regenerated cellulose such as viscose.

The laundering processes of the present invention include the large scale and small scale (e.g. domestic) cleaning of textiles. Preferably, the processes are domestic.

It is particularly preferable that the protected thiol group comprises an isothiouronium group. While such polymers terminated with such groups have previously been used for the treatment of wool and blends of wool with other materials, it is surprising that they are effective in the treatment of non-keratinaceous materials such as cotton and other cellulosic fibres in a domestic laundry process.

It is believed that a further advantage of the preferred materials is that their ability to take on a positive charge gives them an affinity for the textile being treated.

Use of an isothiouronium containing polymer, or other thiol group protected by a suitably labile leaving group, in a composition which further comprises a textile compatible carrier is therefore believed to reduce fibre damage and/or improve the dimensional stability of cellulosic textiles following domestic laundry.

### Detailed Description of the Invention

For illustrative purposes, the compositions of the present invention will be further described below with reference to those comprising at least one polymer comprising at least one isothiouronium group.

The isothiouronium group fulfils the requirements of a suitable protecting group as it is labile under conditions of pH and temperature found in domestic laundry. Typical conditions encountered are a pH from 8 to 11 and a temperature of 10 to 80° Celsius. Slightly higher temperatures are encountered under some ironing and domestic drying conditions. It is preferable that the protecting group is sufficiently labile that it leaves when the polymer is exposed to a pH of above 8 at a temperature of below 50 Celsius.

Isothiouronium salts are advantageous in that they can be heat cured, for example by a domestic ironing or tumble drying procedure. If used, heat curing is preferably carried out at a temperature in the range of from 50 to 100°C, more preferably from 80 to 100°C.

The polymer is preferably present in the textile care composition in a sufficient quantity to give an amount of 0.0005% to 5% by weight on the textile based on the weight of the textile, more preferably 0.001% to 2% by weight on textile.

Preferably, the polymers have a weight average mean molecular weight of from 300 to 1,000,000 Dalton.

### Polymers:

As explained above, the polymers of the invention undergo elimination under mildly alkaline conditions, such as those encountered during a domestic laundering process, to produce thiol intermediates which cross link. Cross-linking can occur by the formation of an insoluble di-sulphide polymer or by other mechanisms as detailed below.

Preferably, the molecular weight of the polymers used is such that any unpleasant odour component associated with the thiol intermediate is not sufficiently volatile to affect the efficacy of the textile care composition.

As will be illustrated hereafter the backbone of the polymer can take several forms. Preferred polymers according to the invention are based on either polypropylene oxide or polyethyleneimine.

Preferably, the protected thiol polymers used in the compositions of the invention have two or more reactive end groups. It had been found preferable to employ polymers with three reactive end groups.

Formula (I) below shows a simplified reaction scheme for generating di-sulphide polymers from protected-thiol polymers based on polypropylene oxide. Formula (II) shows a simplified reaction scheme for the production of protected-thiol polymers based on a polyethyleneimine.

As can be seen from the illustrative Formula 1 the molecules of that series of embodiments contain a number of linked backbones (in this case three) comprising polypropylene oxide (PPO), a linker group towards the free end of the backbone and the protected thiol group at the terminal. At high pH the urea leaves the protected thiol group and the thiol groups cross-link. It would be expected that this cross-linking would not happen in the highly ordered manner shown for illustration below, but would involve more than two molecules, so as to form large and complex structures.

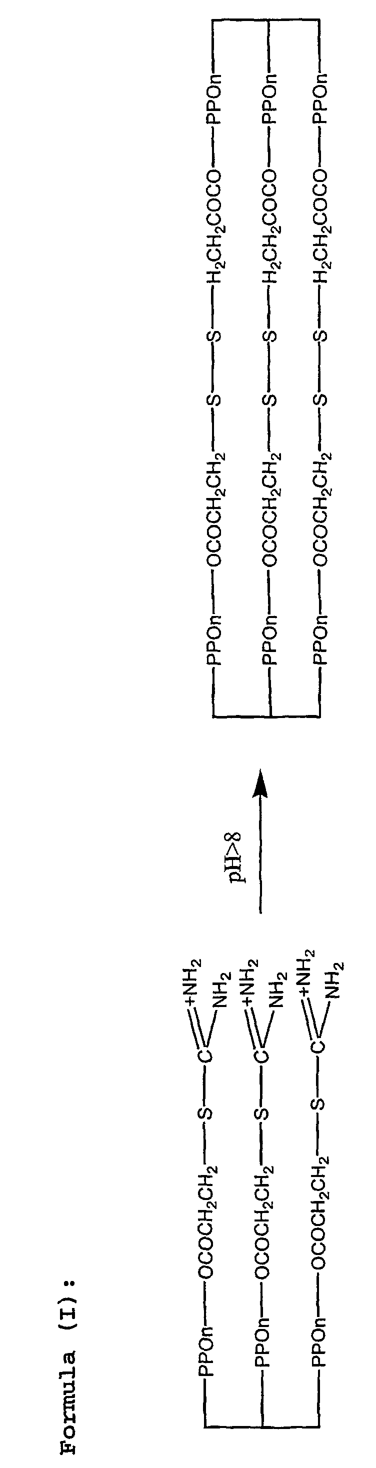

While it is preferred that the protected thiol is in a terminal position (as in Formula I above) it is possible to produce polymers in which the protected thiol is non-terminal (as in Formula II). By way of example, Formula II shows how a protected thiol group may be introduced into a polyethyleneimine by reaction with chloropropionyl chloride followed by thiourea.

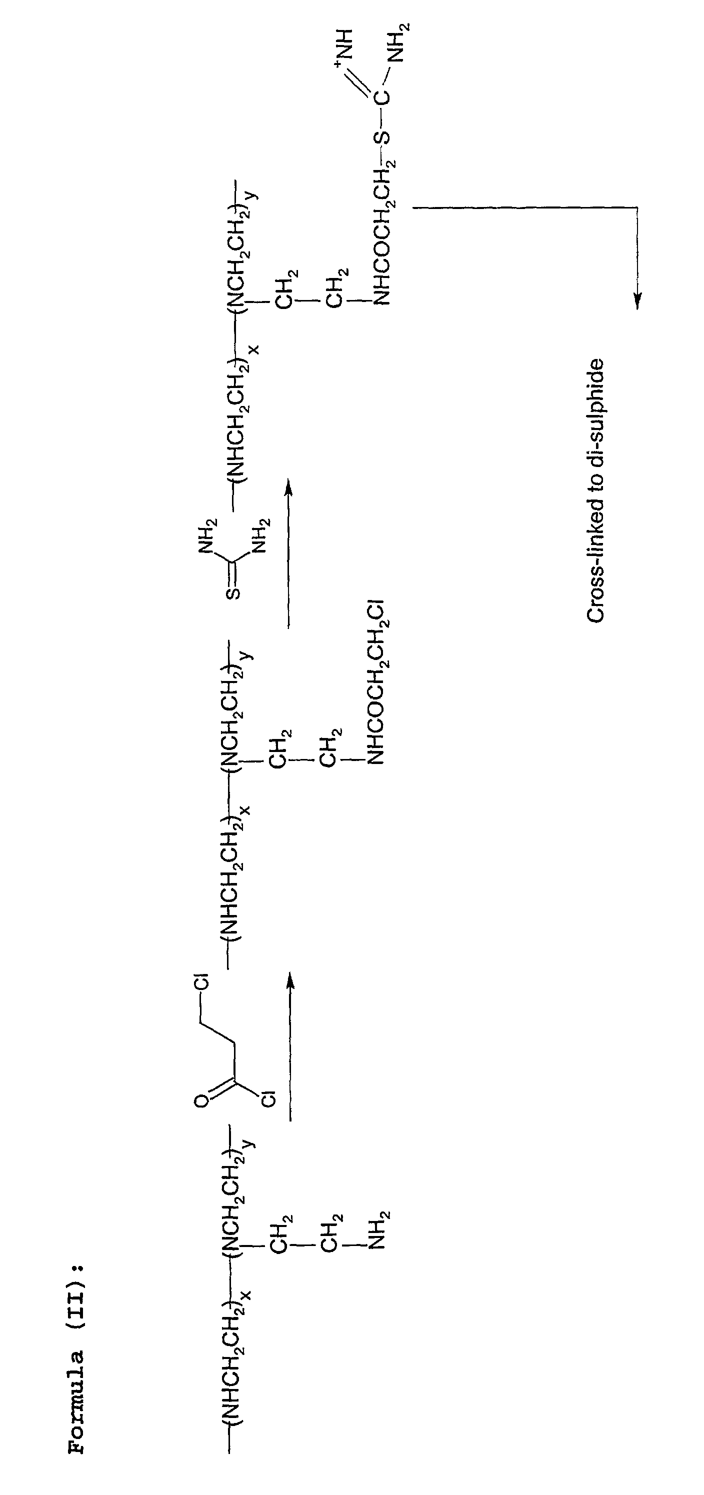

In both the embodiments described above reaction with hydroxyl anions leads to elimination of urea from the protected thiol. This forms a reactive thiol group which reacts with a similar thiol group to form a di-sulphide (S-S-) bridge. As will be described in more detail below, it is also possible for the thiol group to react with other, nonthiol, groups once the protecting group has been removed.

Details of other commercially available polymers which can act as backbones for the isothiouronium containing polymers are as follows:

### Poly (diallylamine)

When poly(diallylamine) is reacted with chloropropionyl chloride and subsequently reacted with thiourea it yields a cationic water soluble reactive polymer with a thiol group protected by a labile urea.

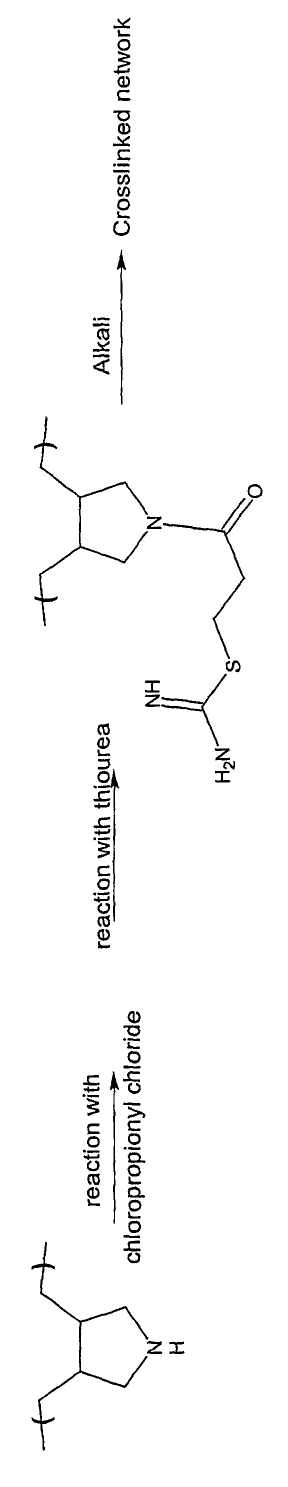

An alternative to this is the copolymerisation of diallyl dimethyl ammonium chloride with diallylamine. The resulting polymer can then be reacted with chloropropionyl chloride and thiourea. This will again yield a cationic polymer.

Poly(diallylamine) polymers can be reacted as above to 50% substitution. The remaining 50% substitution can be a reaction with epichlorohydrin to form a reactive azetidinium group. This provides an example of a polymer in which not all of the reactive groups are a protected thiol. When placed in water at pH>8, isothiouronium would eliminate to a thiol, the thiol would then react with the azetidinium group forming a sulphide cross link. This is an example of a cross-linking reaction other than of the '-S-S-' variety.

Other alternative embodiments are envisaged in which the composition for use in the method of the invention comprises two separate polymers one or both of which comprise isothiouronium reactive groups.

In a particularly preferred embodiment the treatment composition comprises both a polymer comprising the protected thiol and an amine or amide-epichlorohydrin resin having one or more azetidinium functional groups. Examples of such azetidinium containing materials are those available as the 'Hercosett' ™ and 'Listrilan' ™ polymers. These are believed to be amine or amide-epichlorohydrin resin having one or more azetidinium functional groups. Kenores ™ polymers are another suitable PAE resins.

### Diethylenetriamine/adipic acid polymers

Polymers based on diethylenetriamine/adipic acid are reacted with chloropropionyl chloride and thiourea to give isothiouronium containing polymers, as shown in the figure below.

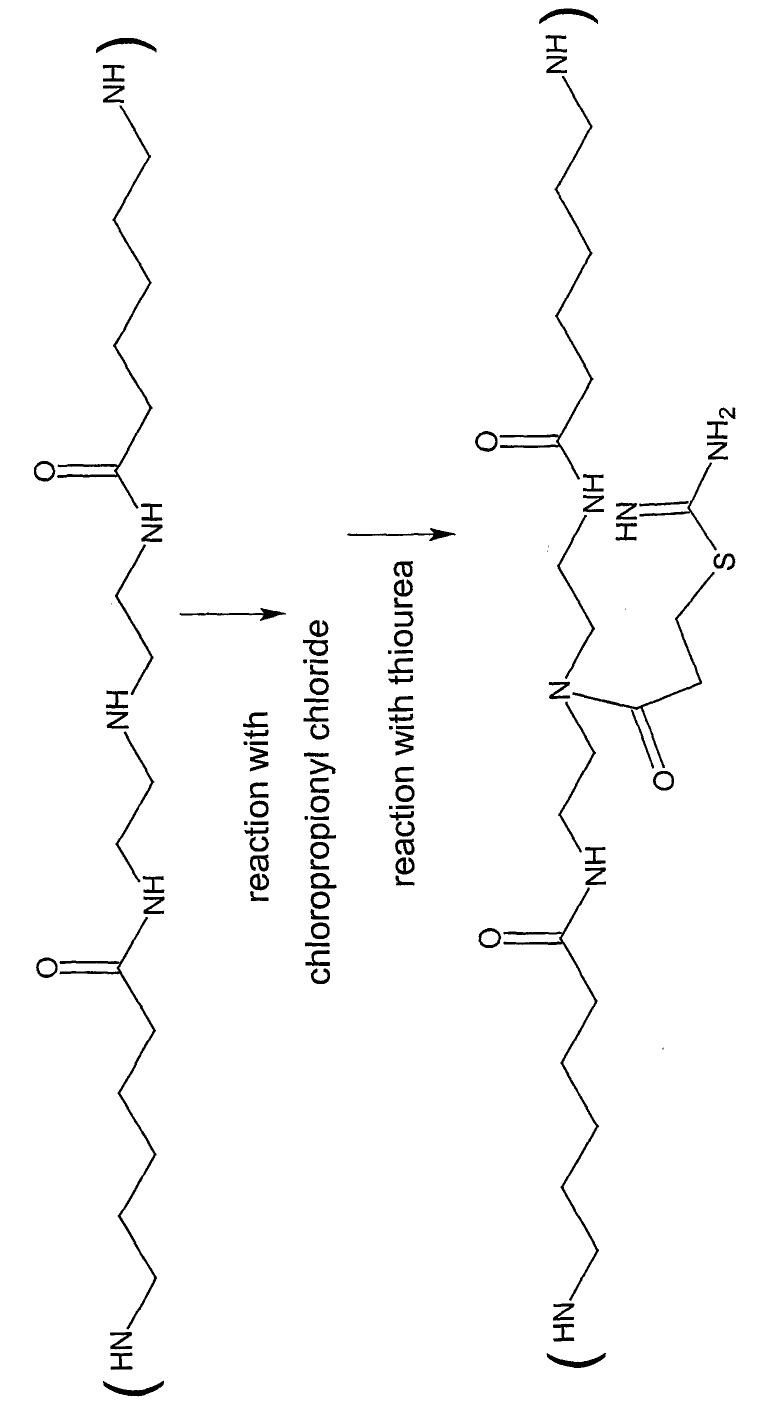

As with the previous examples, this polymer can also be formulated with azetidinium containing polymer for enhanced cross-linking ability.

### Amino functional dendrimers

Embodiments of the invention are not limited to polymers having a relatively small number of terminal or mid chain protected groups. Dendrimers containing a multiplicity of amino groups can be reacted with chloropropionyl chloride and thiourea to give isothiouronium polymers. Suitable starting molecules include Lupasols, ™ (ex-BASF) StarBurst ™ (ex-Dow) or DAB-Am ™ (ex-DSM) molecules.

The backbone of the polymer can be a carbohydrate or carbohydrate derivative as shown below.

### Chitosan

Chitosan can be reacted with chloropropionyl chloride and then with thiourea to give a water soluble cationic product having pendant isothiouronium groups.

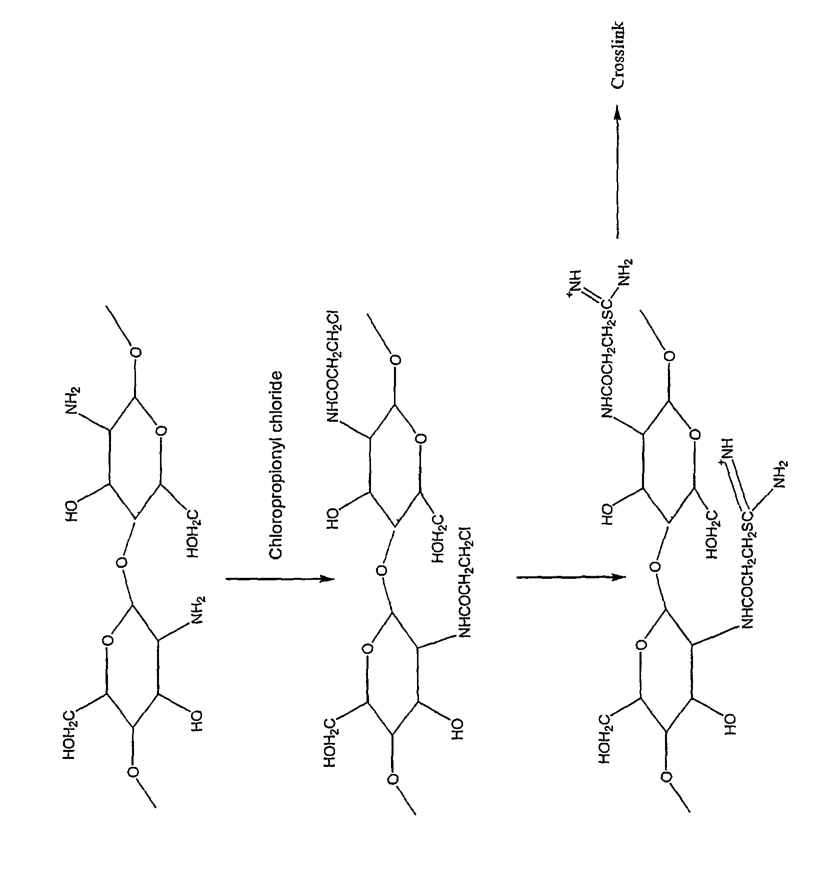

It is not necessary to synthesize the polymers from amines as described above.

### Reaction of azetidinium with thiourea

The reaction of azetidinium polymers with thiourea occurs rapidly to produce isothiouronium groups. These can be mid-chain or terminal. Alkali treatment of the polymers will then cause cross-linking reactions.

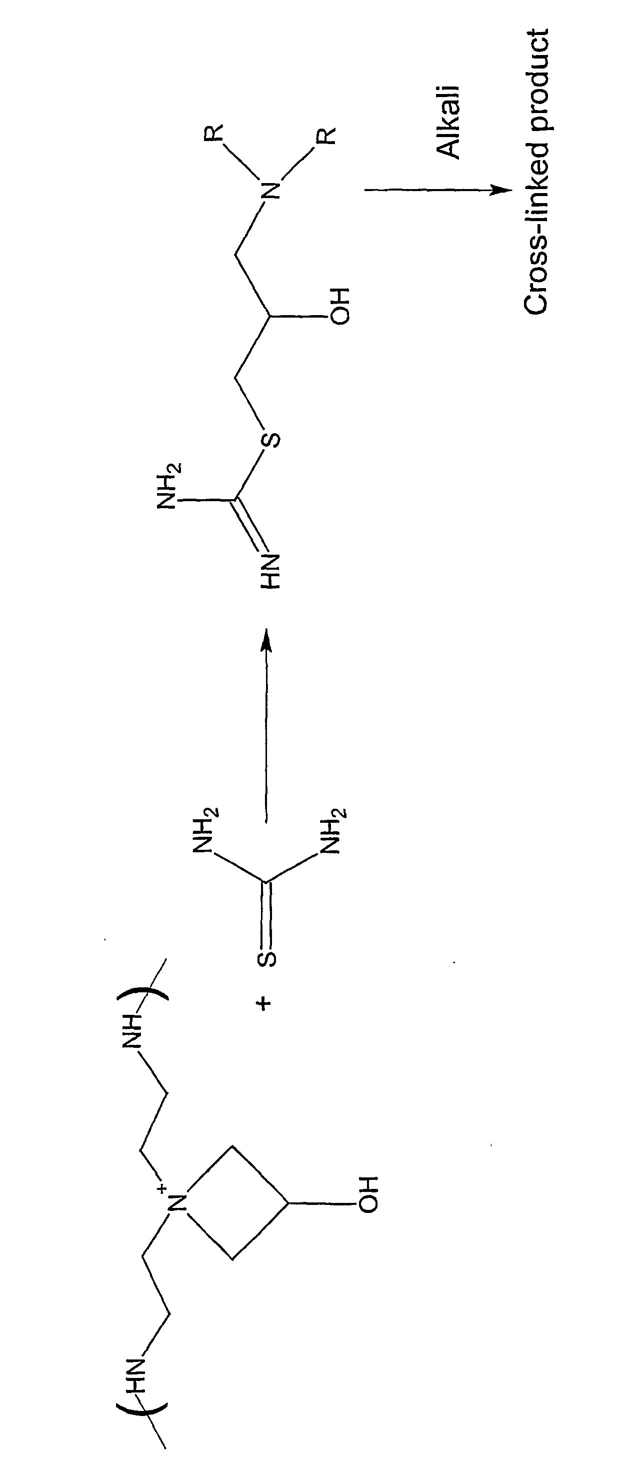

Preferred polymers are those based on the polyamidoamine condensation products of diethylenetriamine and adipic acid. These materials are reacted with epichlorohydrin to give the polyamidoamine/epichlorohydrin ('PAE') polymer. Azetidinium groups are introduced into the polymer by cyclisation of chlorohydrin groups to give the azetidinium ring. Examples of such materials are available as the 'Hercosett' ™, Kenores and 'Listrilan' ™ polymers.

### Poly (vinyl alcohol)

PVA can be reacted with chloropropionyl chloride and then reacted with thiourea to give a cationic water soluble polymer with a labile urea residue. As in the previous descriptions the labile urea may be mid-chain or terminal.

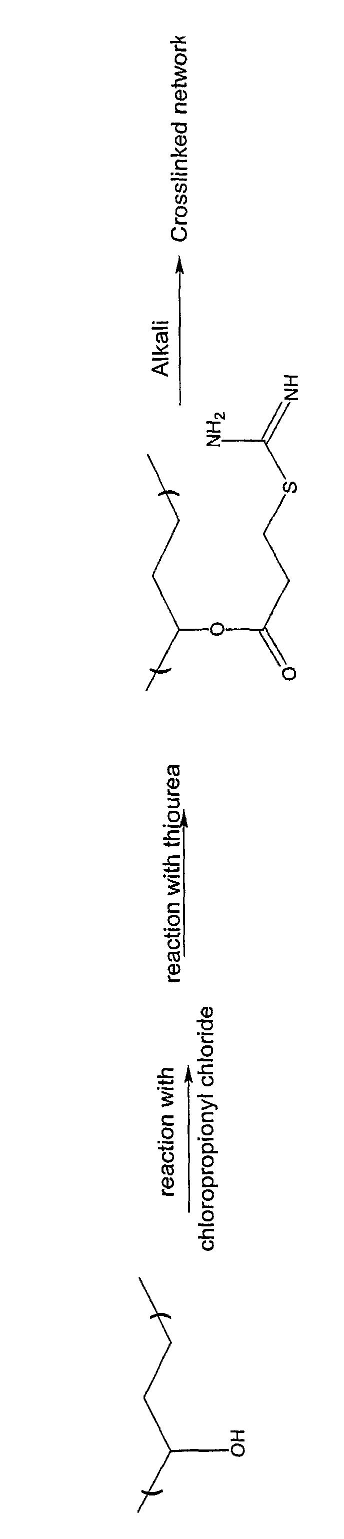

Other polymers can be employed.

### Bunte salt/ isothiouronium derivative of 4,4'aminostilbene-2,2'-disulphonic acid (DAS)

4,4'aminostilbene-2,2'-disulphonic acid (DAS) can be reacted with cyanuric chloride, then with chloroethylamine hydrochloride followed by (typically two moles of) sodium thiosulphate. The remaining chlorine atoms are reacted with thiourea to give isothiouronium groups

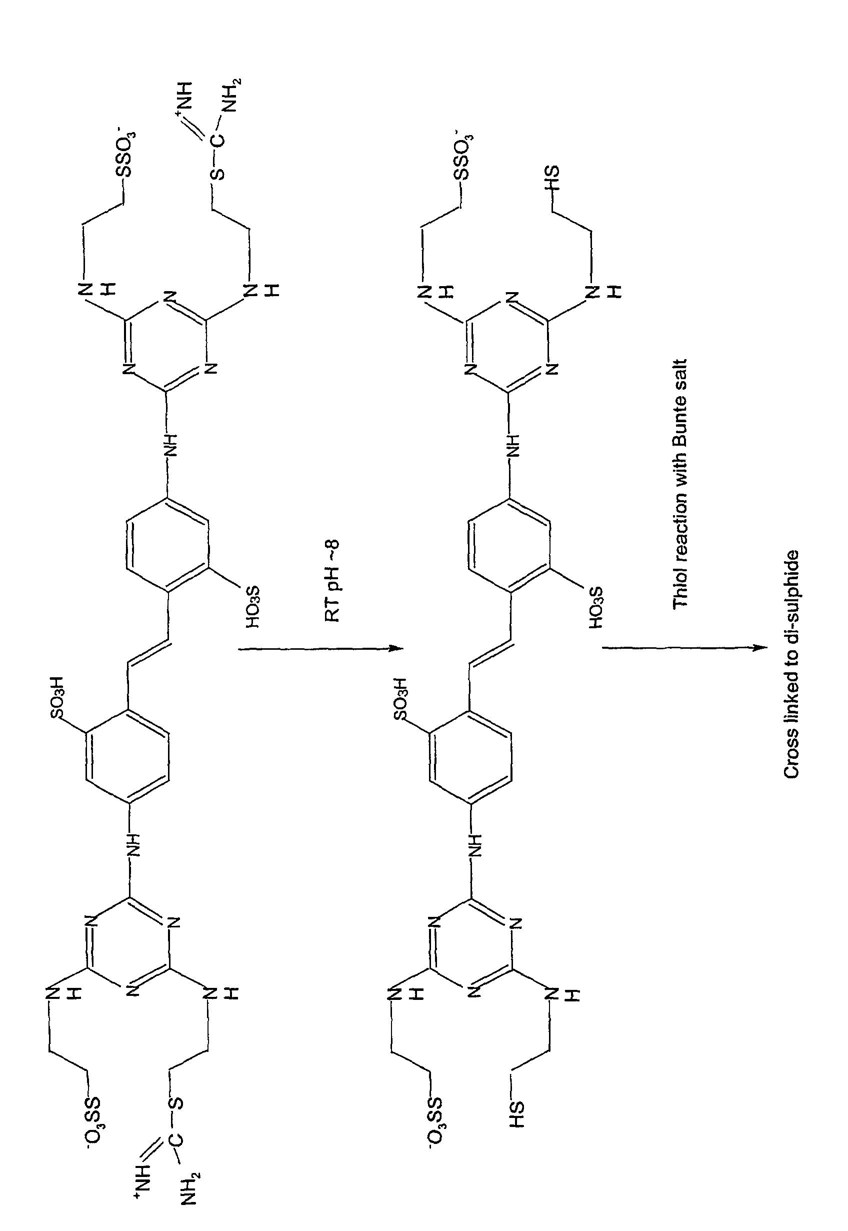

At pH 8 and above, elimination to thiol occurs, followed cross-linking reaction between the thiosulphate groups and the reactive thiol.

### Carriers:

In the context of the present invention the term "textile compatible carrier" is a component which can assist in the interaction of the first component with the textile. The carrier can also provide benefits in addition to those provided by the first component e.g. softening, cleaning etc. The carrier may be a water or a detergent-active compound or a textile softener or conditioning compound or other suitable detergent or textile treatment agent.

If the composition of the invention is to be used in a laundry process as part of a conventional textile treatment product, such as a detergent composition, the textile-compatible carrier will typically be a detergent-active compound. Whereas, if the textile treatment product is a rinse conditioner, the textile-compatible carrier will be a textile softening and/or conditioning compound.

If the composition of the invention is to be used before, or after, the laundry process it may be in the form of a spray or foaming product.

The polymer is preferably used to treat the textile in the rinse cycle of a laundering process. The rinse cycle preferably follows the treatment of the textile with a detergent composition.

### Detergent Active Compounds:

If the composition of the present invention is in the form of a detergent composition, the textile-compatible carrier may be chosen from soap and non-soap anionic, cationic, nonionic, amphoteric and zwitterionic detergent active compounds, and mixtures thereof.

Many suitable detergent active compounds are available and are fully described in the literature, for example, in "Surface-Active Agents and Detergents", Volumes I and II, by Schwartz, Perry and Berch.

The preferred textile-compatible carriers that can be used are soaps and synthetic non-soap anionic and nonionic compounds.

Anionic surfactants are well-known to those skilled in the art. Examples include alkylbenzene sulphonates, particularly linear alkylbenzene sulphonates having an alkyl chain length of C₈-C₁₅; primary and secondary alkylsulphates, particularly C₈-C₁₅ primary alkyl sulphates; alkyl ether sulphates; olefin sulphonates; alkyl xylene sulphonates; dialkyl sulphosuccinates; and fatty acid ester sulphonates. Sodium salts are generally preferred.

Nonionic surfactants that may be used include the primary and secondary alcohol ethoxylates, especially the C₈-C₂₀ aliphatic alcohols ethoxylated with an average of from 1 to 20 moles of ethylene oxide per mole of alcohol, and more especially the C₁₀-C₁₅ primary and secondary aliphatic alcohols ethoxylated with an average of from 1 to 10 moles of ethylene oxide per mole of alcohol. Non-ethoxylated nonionic surfactants include alkylpolyglycosides, glycerol monoethers, and polyhydroxyamides (glucamide).

Cationic surfactants that may be used include quaternary ammonium salts of the general formula R₁R₂R₃R₄N⁺ X⁻ wherein the R groups are independently hydrocarbyl chains of C₁-C₂₂ length, typically alkyl, hydroxyalkyl or ethoxylated alkyl groups, and X is a solubilising cation (for example, compounds in which R₁ is a C₈-C₂₂ alkyl group, preferably a C₈-C₁₀ or C₁₂-C₁₄ alkyl group, R₂ is a methyl group, and R₃ and R₄, which may be the same or different, are methyl or hydroxyethyl groups); and cationic esters (for example, choline esters) and pyridinium salts.

The total quantity of detergent surfactant in the composition is suitably from 0.1 to 60 wt% e.g. 0.5-55 wt%, such as 5-50wt%.

Preferably, the quantity of anionic surfactant (when present) is in the range of from 1 to 50% by weight of the total composition. More preferably, the quantity of anionic surfactant is in the range of from 3 to 35% by weight, e.g. 5 to 30% by weight.

Preferably, the quantity of nonionic surfactant when present is in the range of from 2 to 25% by weight, more preferably from 5 to 20% by weight.

Amphoteric surfactants may also be used, for example amine oxides or betaines.

### Builders:

The compositions may suitably contain from 10 to 70%, preferably from 15 to 70% by weight, of detergency builder. Preferably, the quantity of builder is in the range of from 15 to 50% by weight.

The detergent composition may contain as builder a crystalline aluminosilicate, preferably an alkali metal aluminosilicate, more preferably a sodium aluminosilicate.

The aluminosilicate may generally be incorporated in amounts of from 10 to 70% by weight (anhydrous basis), preferably from 25 to 50%. Aluminosilicates are materials having the general formula:

0.8-1.5 M₂O. Al₂O₃. 0.8-6 SiO₂

where M is a monovalent cation, preferably sodium. These materials contain some bound water and are required to have a calcium ion exchange capacity of at least 50 mg CaO/g. The preferred sodium aluminosilicates contain 1.5-3.5 SiO₂ units in the formula above. They can be prepared readily by reaction between sodium silicate and sodium aluminate, as amply described in the literature.

### Textile Softening and/or Conditioner Compounds:

If the composition of the present invention is in the form of a textile conditioner composition, the textile-compatible carrier will be a textile softening and/or conditioning compound (hereinafter referred to as "textile softening compound"), which may be a cationic or nonionic compound.

The softening and/or conditioning compounds may be water insoluble quaternary ammonium compounds. The compounds may be present in amounts of up to 8% by weight (based on the total amount of the composition) in which case the compositions are considered dilute, or at levels from 8% to about 50% by weight, in which case the compositions are considered concentrates.

Compositions suitable for delivery during the rinse cycle may also be delivered to the textile in the tumble dryer if used in a suitable form. Thus, another product form is a composition (for example, a paste) suitable for coating onto, and delivery from, a substrate e.g. a flexible sheet or sponge or a suitable dispenser during a tumble dryer cycle.

Suitable cationic textile softening compounds are substantially water-insoluble quaternary ammonium materials comprising a single alkyl or alkenyl long chain having an average chain length greater than or equal to C₂₀. More preferably, softening compounds comprise a polar head group and two alkyl or alkenyl chains having an average chain length greater than or equal to C₁₄. Preferably the textile softening compounds have two, long-chain, alkyl or alkenyl chains each having an average chain length greater than or equal to C₁₆.

Most preferably at least 50% of the long chain alkyl or alkenyl groups have a chain length of C₁₈ or above. It is preferred if the long chain alkyl or alkenyl groups of the textile softening compound are predominantly linear.

Quaternary ammonium compounds having two long-chain aliphatic groups, for example, distearyldimethyl ammonium chloride and di(hardened tallow alkyl) dimethyl ammonium chloride, are widely used in commercially available rinse conditioner compositions. Other examples of these cationic compounds are to be found in "Surface-Active Agents and Detergents", Volumes I and II, by Schwartz, Perry and Berch. Any of the conventional types of such compounds may be used in the compositions of the present invention.

The textile softening compounds are preferably compounds that provide excellent softening, and are characterised by a chain melting Lβ to Lα transition temperature greater than 25°C, preferably greater than 35°C, most preferably greater than 45°C. This Lβ to Lα transition can be measured by DSC as defined in "Handbook of Lipid Bilayers", D Marsh, CRC Press, Boca Raton, Florida, 1990 (pages 137 and 337).

Substantially water-insoluble textile softening compounds are defined as textile softening compounds having a solubility of less than 1 x 10⁻³ wt % in demineralised water at 20°C. Preferably the textile softening compounds have a solubility of less than 1 x 10⁻⁴ wt%, more preferably less than 1 x 10⁻⁸ to 1 x 10⁻⁶ wt%.

Especially preferred are cationic textile softening compounds that are water-insoluble quaternary ammonium materials having two C₁₂₋₂₂ alkyl or alkenyl groups connected to the molecule via at least one ester link, preferably two ester links. An especially preferred ester-linked quaternary ammonium material can be represented by the formula III: 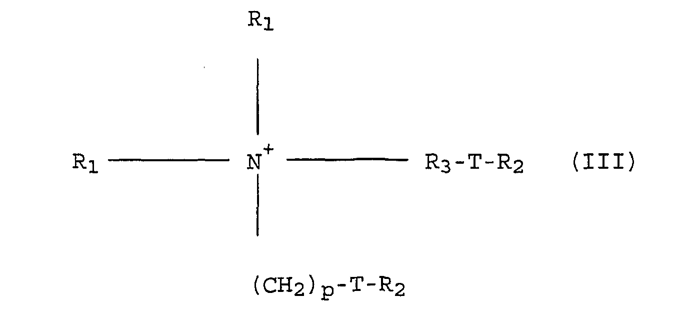 wherein each R₁ group is independently selected from C₁₋₄ alkyl or hydroxyalkyl groups or C₂₋₄ alkenyl groups; each R₂ group is independently selected from C₈₋₂₈ alkyl or alkenyl groups; and wherein R₃ is a linear or branched alkylene group of 1 to 5 carbon atoms, T is 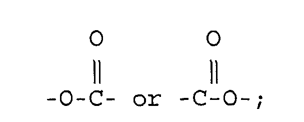 and *p* is 0 or is an integer from 1 to 5.

Di(tallowoxyloxyethyl) dimethyl ammonium chloride and/or its hardened tallow analogue is especially preferred of the compounds of formula (II).

A second preferred type of quaternary ammonium material can be represented by the formula (IV): 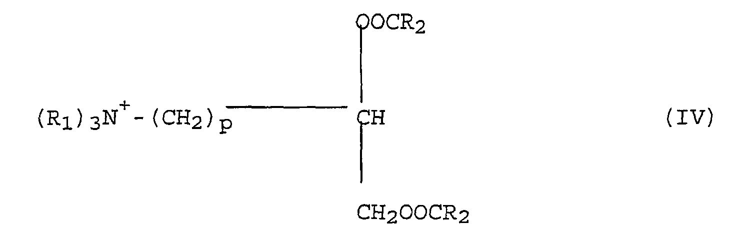 wherein R₁, *p* and R₂ are as defined above.

It is advantageous if the quaternary ammonium material is biologically biodegradable.

Preferred materials of this class such as 1,2-bis(hardened tallowoyloxy)-3-trimethylammonium propane chloride and their methods of preparation are, for example, described in US 4 137 180 (Lever Brothers Co). Preferably these materials comprise small amounts of the corresponding monoester as described in US 4 137 180, for example, 1-hardened tallowoyloxy-2-hydroxy-3-trimethylammonium propane chloride.

Other useful cationic softening agents are alkyl pyridinium salts and substituted imidazoline species. Also useful are primary, secondary and tertiary amines and the condensation products of fatty acids with alkylpolyamines.

The compositions may alternatively or additionally contain water-soluble cationic textile softeners, as described in GB 2 039 556B (Unilever).

The compositions may comprise a cationic textile softening compound and an oil, for example as disclosed in EP-A-0829531.

The compositions may alternatively or additionally contain nonionic textile softening agents such as lanolin and derivatives thereof.

Lecithins are also suitable softening compounds.

Nonionic softeners include Lβ phase forming sugar esters (as described in M Hato et al Langmuir 12, 1659, 1666, (1996)) and related materials such as glycerol monostearate or sorbitan esters. Often these materials are used in conjunction with cationic materials to assist deposition (see, for example, GB 2 202 244). Silicones are used in a similar way as a co-softener with a cationic softener in rinse treatments (see, for example, GB 1 549 180).

The compositions may also suitably contain a nonionic stabilising agent. Suitable nonionic stabilising agents are linear C₈ to C₂₂ alcohols alkoxylated with 10 to 20 moles of alkylene oxide, C₁₀ to C₂₀ alcohols, or mixtures thereof.

Advantageously the nonionic stabilising agent is a linear C₈ to C₂₂ alcohol alkoxylated with 10 to 20 moles of alkylene oxide. Preferably, the level of nonionic stabiliser is within the range from 0.1 to 10% by weight, more preferably from 0.5 to 5% by weight, most preferably from 1 to 4% by weight. The mole ratio of the quaternary ammonium compound and/or other cationic softening agent to the nonionic stabilising agent is suitably within the range from 40:1 to about 1:1, preferably within the range from 18:1 to about 3:1.

The composition can also contain fatty acids, for example C₈ to C₂₄ alkyl or alkenyl monocarboxylic acids or polymers thereof. Preferably saturated fatty acids are used, in particular, hardened tallow C₁₆ to C₁₈ fatty acids. Preferably the fatty acid is non-saponified, more preferably the fatty acid is free, for example oleic acid, lauric acid or tallow fatty acid. The level of fatty acid material is preferably more than 0.1% by weight, more preferably more than 0.2% by weight. Concentrated compositions may comprise from 0.5 to 20% by weight of fatty acid, more preferably 1% to 10% by weight. The weight ratio of quaternary ammonium material or other cationic softening agent to fatty acid material is preferably from 10:1 to 1:10.

### Textile Treatment Products

The composition of the invention may be in the form of a liquid, solid (e.g. powder or tablet), a gel or paste, spray, stick or a foam or mousse. Examples include a soaking product, a rinse treatment (e.g. conditioner or finisher) or a main-wash product. The composition may also be applied to a substrate e.g. a flexible sheet or used in a dispenser which can be used in the wash cycle, rinse cycle or during the dryer cycle.

Liquid compositions may also include an agent which produces a pearlescent appearance, e.g. an organic pearlising compound such as ethylene glycol distearate, or inorganic pearlising pigments such as microfine mica or titanium dioxide (TiO₂) coated mica.

Liquid compositions may be in the form of emulsions or emulsion precursors thereof.

The composition of the invention may further comprise a silicone component. It is preferred if the silicone component is a dimethylpolysiloxane with amino alkyl groups.

It may be used in the context of the present invention as an emulsion in water.

It is preferred if the silicone component is present in a ratio of first component: silicone of from 1:1 to 30:1, preferably 1:1 to 20:1, more preferably 2:1 to 20:1 and most preferably 5:1 to 15:1.

Silicone suitable for use in textile conditioning compositions include predominately linear polydialkylsiloxanes, e.g. polydimethylsiloxanes or aminosilicones containing amine-functionalised side chains.

Composition may comprise soil release polymers such as block copolymers of polyethylene oxide and terephthalate.

Other optional ingredients include emulsifiers, electrolytes (for example, sodium chloride or calcium chloride) preferably in the range from 0.01 to 5% by weight, pH buffering agents, and perfumes (preferably from 0.1 to 5% by weight).

Further optional ingredients include non-aqueous solvents, perfume carriers, fluorescers, colourants, hydrotropes, antifoaming agents, antiredeposition agents, enzymes, optical brightening agents, opacifiers, dye transfer inhibitors.

In addition, compositions may comprise one or more of anti-shrinking agents, anti-wrinkle agents, anti-spotting agents, germicides, fungicides, anti-oxidants, UV absorbers (sunscreens), heavy metal sequestrants, chlorine scavengers, dye fixatives, anti-corrosion agents, drape imparting agents, antistatic agents and ironing aids. The lists of optional components are not intended to be exhaustive.

The invention will now be described by way of example only and with reference to the following non-limiting examples.

### EXAMPLES:

### Example 1: Method of preparation of Voranol iso-thiouronium compounds

Voranol CP3055 ™ (ex-Dow Chemicals) (29.4g, 0.01moles, mw 2940) was dissolved in toluene 50ml. To this was added 3-bromopropionic acid (4.95g, 3.3 equivalents ex Aldrich) and p-toluene sulphonic acid (1g) to act as an acid catalyst. The solution was stirred by means of a magnetic follower on a hot plate. The solution was refluxed with a Dean and Stark distillation trap. After 1.5 hours no more water was seen to azeotrope from the reaction vessel into the side arm. The reaction was stopped and allowed to cool.

The toluene/ Voranol solution was shaken with solution of sodium bicarbonate (10g/l). This was repeated five times, until the solution was seen not to effervesce from the evolution of carbon dioxide gas and the water was at a neutral pH. The two phases were separated and the toluene was removed from the Voranol compound by rotary evaporation. After drying in vacuo over calcium chloride the yield was 29.28g (theoretical yield is 33.44g). The losses were mainly due to the highly viscous sticky nature of the polymer which adhered to glassware. A pale yellow liquid product was obtained.

FT-IR analysis confirmed the presence of an ester group by showing a new peak at 1737cm⁻¹

The product from the previous step was dissolved in ethanol (50ml). To this solution was added thiourea (2.28g, 0.03m ex Aldrich). This solution was then refluxed for six hours and cooled. The product was not isolated since the thick viscous nature of the pure product was inconvenient to handle.

Addition of the compound to an aqueous solution at a pH below 4 gave a water-soluble product. At pH above pH8, the product is converted from the isothiouronium to produce a thiol group. This step results in the precipitation of the polymer and an opaque, cloudy solution was observed.

Addition of the compound to a solution containing an anionic compound e.g. sodium dodecyl sulphate under acidic conditions leads to the precipitation of a white sticky mass.

The formation of a thiol was determined by the use of Ellmans reagent, which reacts with thiol compounds forming an orange coloured solution. The formation and use of Ellmans reagent is described in Practical Protein Chemistry, A Darbre, Wiley Interscience, New York 1970.

Isolation of a small quantity of isothiouronium polymer for FT-IR analysis confirmed the structure by the presence of new peaks correlating to the structure of isothiouronium groups.

### Example 2: Method of preparation of Jeffamine isothiouronium compounds

Jeffamine T-3000 ™ (ex-Huntsman Corp) (30g, 0.01m mw~3000) was dissolved in acetone (50ml) and cooled to below 5°C with an ice bath. Sodium carbonate (2g) was added to the solution with stirring. 3-Chloropropionyl chloride (4.19g, 3.3 equivalents) was dripped into the solution of Jeffamine T-3000 over a period of one hour. The temperature of the solution was kept below 5°C. After the addition of the acid chloride, the temperature was allowed to rise to room temperature over a period of one hour. The solution was then filtered to remove the solid sodium carbonate. The acetone was removed on a rotary evaporator. The solution was then washed five times with a sodium bicarbonate solution (10g/l) until the washings were neutral. The product was then separated from the aqueous solution using a separating funnel and dried over silica in vacuo.

Yield 26.21g (theoretical 33.69). Losses were due to the highly viscous sticky nature of the polymer.

FT-IR analysis confirmed an amide peak at 1637cm⁻¹.

The product from the previous step was dissolved in ethanol (50ml) and thiourea (2.28g, 0.03m, ex Aldrich) was added. The solution was refluxed for six hours. After this time the solution was allowed to cool. The product was not isolated since the thick viscous nature of the pure product was inconvenient to handle.

Addition of the compound to a solution containing an anionic compound e.g. sodium dodecyl sulphate under acidic conditions lead to the precipitation of a white sticky mass.

Addition of the compound to an aqueous solution at a pH below 4 gave a water-soluble product. At pH above pH8, the product is converted from the isothiouronium to produce a thiol group. This step results in the precipitation of the polymer and an opaque, cloudy solution was observed.

The formation of a thiol was determined by the use of Ellmans reagent which reacts with thiol compounds forming an orange coloured solution.

Isolation of a small quantity of isothiouronium polymer for FT-IR analysis confirmed the structure by the presence of new peaks correlating to the structure of isothiouronium groups.

### Example 3: Method of preparation of Lupasol isothiouronium compounds

Lupasol G-20 ™ (ex-BASF) (39g, 0.03moles was dissolved in ethanol (50ml) and cooled to below 5°C with an ice bath.

Sodium carbonate (3g) was added to the solution with stirring. 3-Chloropropionyl chloride (14.22g, 0.09moles, 3 equivalents) was dripped into the Lupasol solution over a period of two hours. The temperature of the solution was kept below 5°C. After the addition of the acid chloride, the temperature was allowed to rise to room temperature over a period of one hour. The solution was then filtered to remove the solid sodium carbonate. The product was not isolated using the methods described previously since the compound is highly water soluble and recovery from an aqueous solution would have proven difficult. A small quantity was isolated for structure confirmation. A new peak was seen at 1637cm⁻¹, indicating the presence of an amide group.

To the alkylated Lupasol G-20 was added thiourea (6.84g, 0.09moles). The solution was refluxed for six hours. A pale yellow product was obtained. After this time the solution was allowed to cool. The product was not isolated since the thick viscous nature of the pure product was inconvenient to handle.

Addition of the product to an aqueous solution at a pH below 4 gave a water soluble product. As pH was increased to above pH8, the product is converted from the isothiouronium to produce a thiol group. This compound does not precipitate since it is inherently water soluble.

The formation of a thiol was determined by the use of Ellmans reagent, which reacts with thiol compounds forming an orange coloured solution.

Isolation of a small quantity of isothiouronium polymer for FT-IR analysis confirmed the structure by the presence of new peaks correlating to the structure of isothiouronium groups.

### Example 4: Experimental procedure to show effect on fibre damage

Experiments were performed with woven cotton which had been pigment printed with red and black stripes. This was laundered in an unmodified AEG Lavamat 50700 ™ washing machine on a cotton cycle at 40°Celsius. The isothiouronium compounds prepared according to the methods described in examples 1-3 were introduced during the rinse cycle. After laundering, the samples were dried at 50° Celsius in a fan oven for 20 minutes.

Samples which had been laundered up to five times were shown to a panel of independent observers for assessment of damage. Samples were observed in a standard light cabinet under D65 illumination. All of the observers were of the opinion that samples treated according to the method of the invention were less damaged than controls. Damage was also measured using the gray scale measurement as detailed in BS1006/A02:1990. Results were as given below in Table 1.

From Table 1 it can be seen that there is a significant reduction in the level of damage as assessed by this method for embodiments of the invention. The least damage occurred when the protected thiol (isothiouronium) polymers were used together with azetidinium-containing polymers.

**Table 1**

| **Grey scale ratings for repeated washings of cloth with control and according to an embodiment of the method of the invention.** | | | | | |
|---|---|---|---|---|---|
| **Treatment** | **Wash 1** | **Wash 2** | **Wash 3** | **Wash 4** | **Wash 5** |
| Untreated | 3 | 2-3 | 2-3 | 2 | 2 |
| 0.5% Voranol isothiouronium (Ex. 1) | 3-4 | 3-4 | 3-4 | 3 | 3 |
| 1.0% Voranol isothiouronium (Ex. 1) | 3-4 | 3-4 | 3-4 | 3-4 | 3 |
| 0.5% Jeffamine isothiouronium (Ex. 2) | 3-4 | 3-4 | 3-4 | 3 | 3 |
| 1.0% Jeffamine isothiouronium (Ex. 2) | 3-4 | 3-4 | 3-4 | 3-4 | 3 |
| 0.5% Lupasol isothiouronium (Ex. 3) | 3 | 3 | 3 | 2-3 | 2-3 |
| 1.0% Lupasol isothiouronium (Ex. 3) | 3 | 3 | 2-3 | 2-3 | 2-3 |
| 0.5% Voranol isothiouronium (Ex. 1) + 0.5% Listrilan azetidinium | 4-5 | 4-5 | 4 | 4 | 4 |
| 1.0% Voranol isothiouronium (Ex. 1) + 1.0% Listrilan azetidinium | 4-5 | 4-5 | 4-5 | 4-5 | 4 |

## Claims

1. A process for the treatment of non-keratinaceous textiles which comprises the step of treating the textiles with a composition comprising:
a) a polymer comprising at least one protected thiol group, wherein the protecting group is labile under domestic washing conditions of pH from 8 to 11 and a temperature of from 10 to 80° Celsius, and
b) a textile compatible carrier,
whereby under said domestic washing conditions, the polymer forms reactive thiol groups which cause covalent cross-linking of the polymer.

2. A process according to Claim 1, wherein the textile comprises cotton or regenerated cellulose.

3. A process according to Claim 1, wherein the composition comprises a detergent active compound.

4. A process according to Claim 1, wherein the composition comprises a textile softening and/or conditioning compound.

5. A process according to Claim 1, wherein the protected thiol comprises a terminal or mid chain isothiouronium group.

6. A process according to Claim 1 wherein the polymer is present in the composition in an amount such that from 0.0005% to 5% by weight on weight of textile is provided.

7. A process according to Claim 1 wherein the composition comprises a polymer having azetidinium groups and/or one or more functional groups capable of forming azetidinium groups.

8. A process according to claim 6 wherein the composition comprises an amine or amide-epichlorohydrin resin having one or more azetidinium functional groups.

9. A process as claimed in Claim 1, wherein the composition is applied to the textile during the rinse cycle of the laundering process.

## Patentansprüche

1. Verfahren zur Behandlung von nicht keratinartigen Textilien, das den Schritt der Behandlung der Textilien mit einer Zusammensetzung umfaßt, umfassend:
a) ein Polymer, das zumindest eine geschützte Thiolgruppe umfaßt, wobei die Schutzgruppe unter Haushaltswaschbedingungen mit pH 8 bis 11 und einer Temperatur von 10 bis 80 °C labil ist, und
b) einen textilkompatiblen Träger;
wobei unter den Haushaltswaschbedingungen das Polymer reaktive Thiolgruppen bildet, die eine kovalente Vernetzung des Polymers verursachen.

2. Verfahren nach Anspruch 1, wobei die Textilie Baumwolle oder regenerierte Cellulose umfaßt.

3. Verfahren nach Anspruch 1, wobei die Zusammensetzung eine reinigungsaktive Verbindung umfaßt.

4. Verfahren nach Anspruch 1, wobei die Zusammensetzung eine textilerweichende und/oder -konditionierende Verbindung umfaßt.

5. Verfahren nach Anspruch 1, wobei das geschützte Thiol eine terminale oder eine mittelkettige Isothiouroniumgruppe umfaßt.

6. Verfahren nach Anspruch 1, wobei das Polymer in der Zusammensetzung in einer solchen Menge enthalten ist, das 0,0005 bis 5 Gew.-% bezogen auf des Gewicht der Textilie bereitgestellt werden.

7. Verfahren nach Anspruch 1, wobei die Zusammensetzung ein Polymer mit Azetidiniumgruppen und/oder eine oder mehrere funktionelle Gruppen, die zur Bildung von Azetidiniumgruppen fähig sind, umfaßt.

8. Verfahren nach Anspruch 6, wobei die Zusammensetzung ein Amin- oder Amid-Epichlorhydrinharz mit einer oder mehreren Azetidinium-funktionellen Gruppen umfaßt.

9. Verfahren nach Anspruch 1, wobei die Zusammensetzung auf die Textilie während des Spülkreislaufes des Waschverfahrens aufgetragen wird.

## Revendications

1. Procédé destiné au traitement de textiles exempts de kératine qui comprend l'étape de traiter les textiles avec une composition comprenant:
a) un polymère comprenant au moins un groupe thiol protégé, dans lequel le groupe protecteur est labile dans des conditions de lavage domestique à un pH de 8 à 11 et à une température de 10 à 80°Celsius, et
b) un support compatible avec les textiles,
moyennant quoi dans lesdites conditions de lavage domestique, le polymère forme des groupes thiol réactifs ce qui provoque la réticulation covalente du polymère.

2. Procédé selon la revendication 1, dans lequel le textile comprend du coton ou de la cellulose régénérée.

3. Procédé selon la revendication 1, dans lequel la composition comprend un composé détersif actif.

4. Procédé selon la revendication 1, dans lequel la composition comprend un composé adoucissant et/ou assouplissant pour textile.

5. Procédé selon la revendication 1, dans lequel le thiol protégé comprend un groupe isothiouronium terminal ou en milieu de chaîne.

6. Procédé selon la revendication 1 dans lequel le polymère est présent dans la composition dans une quantité telle que de 0,0005 % à 5 % en poids/poids de textile est fourni.

7. Procédé selon la revendication 1 dans lequel la composition comprend un polymère ayant des groupes azétidinium et/ou un ou plusieurs groupes fonctionnels capables de former des groupes azétidinium.

8. Procédé selon la revendication 6 dans lequel la composition comprend une résine amine ou amide-épichlorohydrine ayant un ou plusieurs groupes fonctionnels azétidinium.

9. Procédé selon la revendication 1, dans lequel la composition est appliquée sur le textile pendant le cycle de rinçage du procédé de blanchissage.
